# EUROPEAN PATENT APPLICATION

(11) **EP 4 311 479 A1**
(43) Date of publication of application: **31.01.2024**
(21) Application number: 22215822.2
(22) Date of filing: 22.12.2022
(51) Int. Cl.: A61B 5/00, A61B 5/11, A61B 5/16

(54) **STATE OF CONSCIOUSNESS ANALYSIS APPARATUS, STATE OF CONSCIOUSNESS ANALYSIS PROGRAM, AND OBSERVATION SYSTEM**

(30) Priority: 25.07.2022 JP 2022118250
(71) Applicant: Cross Sync, Inc., Yokohama-shi, Kanagawa 236-0004 (JP)
(72) Inventor: NAMBU, Yuma, Kanagawa, 236-0004 (JP); TABATA, Atsushi, Kanagawa, 236-0004 (JP)
(74) Representative: Plougmann Vingtoft a/s

(57) **Abstract**

A state of consciousness analysis apparatus (20) is provided with: an image acquisition unit (36) that acquires image data (52) expressing a time series of images obtained by capturing a subject (12) on a bed (16) and an area around the subject (12); an object detection unit (38) that performs an object detection process on the acquired image data (52) to detect a temporal change in eye opening/closing of the subject (12); and a state estimation unit (40) that uses at least an eye opening/closing feature relating to the temporal change in eye opening/closing to estimate the state of consciousness of the subject (12).

## Description

### BACKGROUND OF THE INVENTION

### Field of the Invention

The present invention relates to a state of consciousness analysis apparatus, a state of consciousness analysis program, and an observation system.

### Description of the Related Art

In recent years, research and development has been carried out in the medical field to analyze biometric information measured from subjects, including patients, and utilize the obtained analysis results for treatment, diagnosis, and the like of the individual or others. For example, a technology for accurately estimating the severity of the condition of a subject on the basis of various information related to the subject is known.

International Publication No. WO 2020/203015 discloses a system for estimating the severity of the condition of a patient by acquiring clinical image data of an imaging area including the bed of the patient taken over time, analyzing movement of the patient or parts of the body of the patient taken in the acquired clinical image data, and calculating, on the basis of the analyzed movement, a score for at least one of oxygen administration or state of consciousness included in an early warning score index. International Publication No. WO 2020/203015 also indicates that whether or not the patient's eyes are open is used as one of the determination conditions.

### SUMMARY OF THE INVENTION

Incidentally, if a subject is in a confused (or delirious) state, including delirium, or is under sedation control, for example, correctly estimating the subject's state of consciousness merely according to whether the eyes are open or closed may be difficult in some cases. In other words, there is considerable room for improvement in the system disclosed in International Publication No. WO 2020/203015 from the standpoint of estimation accuracy.

The present invention has been created in light of problems like the above, and provides a state of consciousness analysis apparatus, a state of consciousness analysis program, and an observation system with which the estimation accuracy regarding a subject's state of consciousness can be improved under a variety of circumstances.

A state of consciousness analysis apparatus according to a first aspect of the present invention is provided with: an image acquisition unit that acquires image data expressing a time series of images obtained by capturing a subject on a bed and an area around the subject; an object detection unit that performs an object detection process on the image data acquired by the image acquisition unit to detect a temporal change in eye opening/closing of the subject; and a state estimation unit that uses at least an eye opening/closing feature relating to the temporal change in eye opening/closing detected by the object detection unit to estimate the state of consciousness of the subject.

A state of consciousness analysis program according to a second aspect of the present invention causes one or multiple computers to execute: an acquiring step of acquiring image data expressing a time series of images obtained by capturing a subject on a bed and an area around the subject; a detecting step of detecting a temporal change in eye opening/closing of the subject on the basis of the acquired image data; and an estimating step of using at least an eye opening/closing feature relating to the detected temporal change in eye opening/closing to estimate the state of consciousness of the subject.

An observation system according to a third aspect of the present invention is provided with: a camera that outputs image data expressing a time series of images obtained by capturing a subject on a bed and an area around the subject; a state of consciousness analysis apparatus that gives an instruction for external notification on a basis of the image data outputted from the camera; and a notification apparatus that produces an external notification in response to the notification instruction from the state of consciousness analysis apparatus, the state of consciousness analysis apparatus being provided with: an image acquisition unit that acquires the image data outputted by the camera; an object detection unit that performs an object detection process on the image data acquired by the image acquisition unit to detect a temporal change in eye opening/closing of the subject; a state estimation unit that uses at least an eye opening/closing feature relating to the temporal change in eye opening/closing detected by the object detection unit to estimate the state of consciousness of the subject; and a notification instruction unit that determines whether an external notification is necessary on the basis of an estimation result obtained by the state estimation unit, and gives a notification instruction in a case where the notification is determined to be necessary.

A storage medium according to a fourth aspect of the present invention is a non-transitory computer-readable storage medium storing a state of consciousness analysis program causing one or multiple computers to execute: an acquiring step of acquiring image data expressing a time series of images obtained by capturing a subject on a bed and an area around the subject; a detecting step of detecting a temporal change in eye opening/closing of the subject on the basis of the acquired image data; and an estimating step of using at least an eye opening/closing feature relating to the detected temporal change in eye opening/closing to estimate the state of consciousness of the subject.

According to the present invention, the estimation accuracy regarding a subject's state of consciousness can be raised further under a variety of circumstances.

The above and other objects, features, and advantages of the present invention will become more apparent from the following description when taken in conjunction with the accompanying drawings in which a preferred embodiment of the present invention is shown by way of illustrative example.

### BRIEF DESCRIPTION OF THE DRAWINGS

FIG. 1 is an overall configuration diagram of an observation system according to an embodiment of the present invention;
FIG. 2 is a diagram illustrating an example of the configuration of the server apparatus in FIG. 1;
FIG. 3 is a diagram illustrating a points table for the Glasgow Coma Scale (GCS), which indicates the severity of consciousness impairment;
FIG. 4 is a basic flowchart related to operations for estimating the state of consciousness;
FIG. 5 is a perspective view illustrating an example inside the room in FIG. 1;
FIG. 6 is a table illustrating an example of determination conditions specified by determination information related to a temporal change in eye opening/closing;
FIG. 7 is a table illustrating an example of determination conditions specified by determination information related to body movement of a subject;
FIG. 8 is a first flowchart illustrating a specific example of estimation operations;
FIG. 9 is a second flowchart illustrating a specific example of estimation operations;
FIG. 10 is a third flowchart illustrating a specific example of estimation operations; and
FIG. 11 is a fourth flowchart illustrating a specific example of estimation operations.

### DESCRIPTION OF THE PREFERRED EMBODIMENT

Hereinafter, an embodiment of the present invention will be described with reference to the attached drawings. To facilitate understanding, like elements and steps in each of the drawings are denoted with like signs wherever possible, and a duplicate description is omitted.

### [Configuration of observation system 10]

### <Overall configuration>

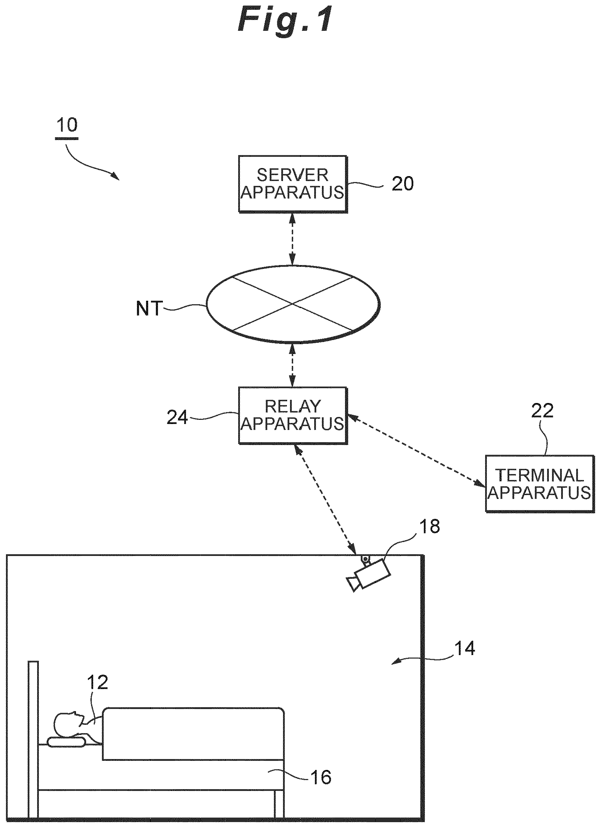

FIG. 1 is an overall configuration diagram of an observation system 10 according to an embodiment of the present invention. The observation system 10 is configured to be able to provide a "watch-over service" for observing (or monitoring) the state of consciousness of a subject 12. In the example illustrated in the drawing, the subject 12 is lying on a bed 16 provided inside a room 14 of a hospital, one's own home, or the like. Specifically, the observation system 10 includes one or multiple cameras 18, a server apparatus 20 (corresponding to a "state of consciousness analysis apparatus"), and one or multiple terminal apparatuses 22 (corresponding to a "notification apparatus").

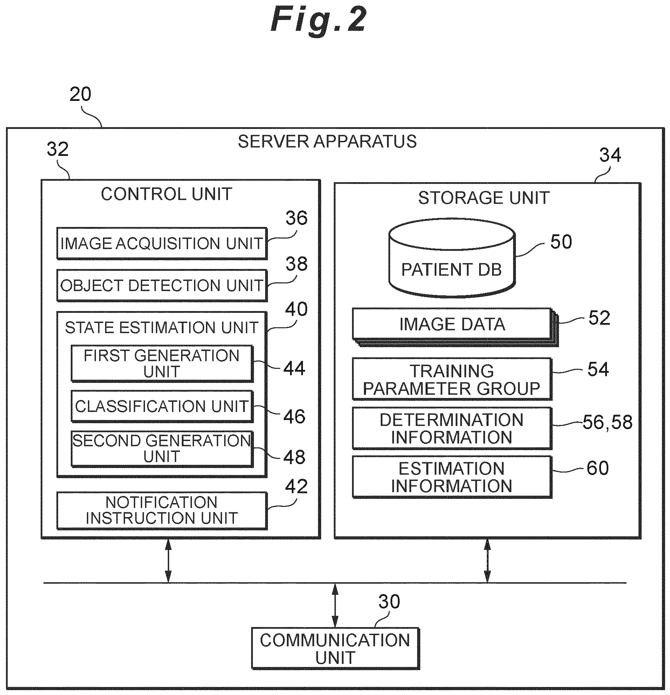

The camera 18 is an image capture apparatus that generates an image signal for each frame taken by capturing the interior of the room 14, and outputs the image signal as image data 52 (FIG. 2) expressing a time series of images. The camera 18 is a visible light camera, an infrared camera, a time-of-flight (ToF) camera, a stereo camera formed by cameras of the same type, or a combination of different types of cameras. The camera 18 is installed in a fixed location such that the bed 16 is contained in the angle of view. The image data 52 can be used to track the location and pose of the subject 12 on the bed 16.

The server apparatus 20 is a computer that provides central control related to the watch-over service described above, and may be of the cloud type or the onpremises type. Although the server apparatus 20 is illustrated as a standalone computer herein, the server apparatus 20 instead may be a computer cluster forming a distributed system.

The terminal apparatus 22 is a computer carried by a user who uses the observation service, and includes output function units (specifically, a display, speaker, and the like) for external notification in a visual or auditory manner. The terminal apparatus 22 may be a smartphone, a tablet, or a personal computer, for example.

The relay apparatus 24 is communication equipment for connecting computers together over a network, and may be a router, a gateway, or a base station, for example. With this arrangement, the camera 18 and the server apparatus 20 are configured to communicate with each other through the relay apparatus 24 and a network NT. Additionally, the server apparatus 20 and the terminal apparatus 22 are configured to communicate with each other through the relay apparatus 24 and the network NT.

### <Configuration of server apparatus 20>

FIG. 2 is a diagram illustrating an example of the configuration of the server apparatus 20 in FIG. 1. Specifically, the server apparatus 20 is a computer including a communication unit 30, a control unit 32, and a storage unit 34.

The communication unit 30 is an interface for transmitting and receiving electrical signals to and from external apparatuses. With this arrangement, the server apparatus 20 can acquire the image data 52 sequentially outputted from the camera 18 and also supply notification data including estimation information 60 generated by the server apparatus 20 itself to the terminal apparatus 22.

The control unit 32 is configured by a processor including a central processing unit (CPU) or a graphics processing unit (GPU). The control unit 32 reads out and executes programs and data stored in the storage unit 34, and thereby functions as an image acquisition unit 36, an object detection unit 38, a state estimation unit 40, and a notification instruction unit 42.

The image acquisition unit 36 acquires the image data 52 expressing a time series of images (that is, frame-by-frame images) obtained by capturing the subject 12 on the bed 16 and the area around the subject 12. The image acquisition unit 36 may acquire the image data 52 received from the camera 18 directly, or read out and acquire the image data 52 previously stored in the storage unit 34.

The object detection unit 38 performs an object detection process on the image data 52 acquired by the image acquisition unit 36 to detect the presence or absence and location of an object in the images. For example, the object detection unit 38 may include a trained neural network on which what is called an object detection model is built, for example. The object detection model may be a "two-stage detector" (for example, Faster R-CNN, Mask R-CNN, or a derivative thereof) in which the region-of-interest (ROI) extractor is provided separately from the object detector, or a "one-stage detector" (for example, YOLO (You Only Look Once), SSD (Single Shot Multibox Detector), M2Det, or a derivative thereof) in which the extractor and the detector are integrated into a single unit.

The objects to be detected are things that may exist inside the room 14, such as human beings, parts of the body, equipment, and the bed 16, for example. Examples of "human beings" include the subject 12 on the bed 16 and a different person (such as a medical personnel member or an attendant, for example) near the bed 16. Examples of "parts of the body" include the eyes, the mouth, the head, the hands, and the feet. Examples of "equipment" include medical equipment installed around the subject 12 and instruments attached to or worn by the subject 12.

The state estimation unit 40 estimates the state of consciousness of the subject 12 on the basis of a detection result from the object detection unit 38. Specifically, the state estimation unit 40 is provided with a first generation unit 44, a classification unit 46, and a second generation unit 48.

The first generation unit 44 generates various features for estimating the state of consciousness of the subject 12 on the basis of the detection result from the object detection unit 38. The features indicating the state of consciousness may be, for example, [1] "qualitative values" that directly indicate the consciousness of the subject 12, such as an alert state, a confused (or delirious) state, a verbally-responsive state, a pain-responsive state, or an unresponsive state, [2] "qualitative values" that indicate conditions with relevance to consciousness, such as a type of blinking (spontaneous/voluntary/reflexive), whether or not the subject 12 is under sedation, and whether or not the subject 12 is under treatment, or [3] "quantitative values" including levels (discrete values) and scores (continuous values). Examples of the features include [1] an "eye opening/closing feature" relating to a temporal change in eye opening/closing of the subject 12, [2] a "movement feature" relating to body movement of the subject 12, or [3] a "response feature" relating to the presence/absence or magnitude of a response from the subject 12 with respect to a specific object.

The eye opening/closing feature may include, for example, [1] a statistic relating to the frequency or speed of blinking within a given determination time, or [2] a statistic indicating the proportion of the eyes-open time or the eyes-closed time (that is, an eyes-open ratio or an eyes-closed ratio) within a given determination time. The determination time may be the most recent time frame going back a prescribed length of time from the present (that is, the time of determination) as a starting point. Examples of the statistics include the value of the mean, the maximum, the minimum, the mode, or the median. Note that the eye opening/closing feature may be [1] obtained from one or both eyes in the case where both eyes of the subject 12 are detected at the same time, or [2] obtained from one detected eye in the case where the other eye is hidden by a bandage or the like.

The movement feature may include, for example, [1] a statistic relating to the velocity, acceleration, or distance of body movement within a given determination time, or [2] a statistic relating to a specific behavior of the subject 12 within a given determination time. The velocity, acceleration, or distance of body movement is calculated using any of various analysis techniques, including optical flow. Examples of the specific behavior include an act in which the subject 12 attempts to touch an instrument attached to or worn on their own body.

The response feature may include, for example, [1] a statistic relating to the velocity, acceleration, or moving distance of a specific object within a given determination time, or [2] a statistic relating to a temporal change in the eye opening/closing feature or the movement feature before and after a specific object moves. The specific object may be a different person (such as a medical personnel member or an attendant, for example) near the bed 16 or a piece of medical equipment installed near the subject 12. For example, the response feature may take a larger value [1] in the case where a person or object approaches the subject 12, or [2] in the case where the subject 12 directs their face or gaze toward an approaching person or object.

The classification unit 46 uses the various features generated by the first generation unit 44 to perform classification into one of a plurality of predetermined levels. Specifically, the plurality of levels may be classified in accordance with one of [1] a consciousness scale, including the Glasgow Coma Scale (GCS), the Japan Coma Scale (JCS), or ACVPU (Alert/Confusion/Verbal/Pain/Unresponsive), [2] a sedation scale, including the Richmond Agitation-Sedation Scale (RASS), [3] an independently defined scale, or [4] any combination of the above scales. As described later, in the GCS, four eye opening levels (E1 to E4), five verbal response levels (V1 to V5), and six motor response levels (M1 to M6) are respectively defined.

In the case where at least the eye opening/closing feature is used, the classification unit 46 may classify the state of consciousness of the subject 12 into one of the plurality of eye opening levels by performing multiple varieties of determination processes differing in the combination of the type of eye opening/closing feature and the determination time length. In this case, a feature is defined as respectively different types of features if at least one of the definition of the value, the number of values, or the method of calculating the feature is different. Also, the determination time length is freely selected in the range from a few seconds to tens of minutes, for example. Specifically, the determination time length is respectively selected from each of [1] a range from a few seconds to tens of seconds for a short-time determination, [2] a range from tens of seconds to a few minutes for a medium-term determination, and [3] a range from a few minutes to tens of minutes for a long-term determination.

In the case where at least the movement feature is used, the classification unit 46 may classify the state of consciousness of the subject 12 into one of the plurality of motor response levels by performing multiple varieties of determination processes differing in the combination of the type of movement feature and the determination time length. In this case, a feature is defined as respectively different types of features if at least one of the definition of the value, the number of values, or the method of calculating the feature is different. Also, the determination time length is freely selected in the range from tens of seconds to tens of minutes, for example.

In the case where at least the response feature is used, the classification unit 46 may estimate the state of consciousness such that the severity is greater to the extent that the response feature is small, or such that the severity is lesser to the extent that the response feature is large. Alternatively, the classification unit 46 may estimate the state of consciousness of the subject 12 by excluding a time frame in which the response feature exceeds a threshold value.

The classification unit 46 may also perform classification according to different rules depending on whether a sedative is administered to the subject 12. The "different rules" means that there is a difference in at least one of [1] the type of feature to be used in a determination process, [2] the threshold value to be used in a determination process, [3] the determination time length, [4] a conditional branch in a determination process, [5] the definition/number of classification levels, [6] the number of times a determination process is executed, [7] the adoption or non-adoption of a determination process, and [8] the order in which determination processes are executed.

The second generation unit 48 generates the estimation information 60 indicating an estimation result obtained via the classification process performed by the classification unit 46, and associates the estimation information 60 with the subject 12.

The notification instruction unit 42 determines whether an external notification is necessary on the basis of the estimation result obtained by the state estimation unit 40, and gives a notification instruction in the case where a notification is determined to be necessary. Specifically, the notification instruction unit 42 causes notification data including the estimation information 60 to be transmitted to a relevant terminal apparatus 22 through the communication unit 30 (FIG. 2).

The storage unit 34 stores programs and data necessary for the control unit 32 to control each element. The storage unit 34 includes a non-transitory and computer-readable storage medium. Here, the computer-readable storage medium may be a portable medium such as a magnetic disk, a read-only memory (ROM), a Compact Disc ROM (CD-ROM), or flash memory, or may be a storage apparatus such as a hard disk drive (HDD) or solid-state drive (SSD) built into a computer system.

In the example illustrated in the drawings, a database (hereinafter referred to as the "patient DB 50") relating to a patient treated as the subject 12 is constructed in the storage unit 34, and in addition, the image data 52, a learning parameter group 54, determination information 56 and 58, and the estimation information 60 are stored in the storage unit 34.

Records forming the patient DB 50 include, for example, [1] a "patient identification (ID)", which is identification information of the patient, [2] "chart information" including physical and diagnostic information about the patient, [3] "network information" about a notification destination, [4] a "date and time of estimation" regarding the state of consciousness, [5] an "estimation result" specified by the estimation information 60, and [6] a notification flag.

The image data 52 is data expressing a time series of images obtained by capturing the subject 12 on the bed 16 and the area around the subject 12. For example, in the case where the camera 18 is a visible-light camera, the image for each frame contains three color channels respectively representing RGB color values. Also, in the case where the camera 18 is a camera unit combining a visible-light camera and a ToF camera, the image for each frame contains four color channels respectively representing RGB color values and depth (D).

The learning parameter group 54 is a set of learning parameters to be used in computations by the object detection unit 38 (more specifically, the object detection model). The training parameter group 54 includes "variable parameters" which are to be updated during learning and "fixed parameters" (also referred to as hyperparameters) which are not to be updated during learning. Examples of the variable parameters include coefficients describing activation functions of nodes and the coupling strength between nodes. Examples of fixed parameters include the number of nodes, the number of intermediate layers, and the kernel size for convolutional operations.

The determination information 56 and 58 is information describing the determination processes for estimating the state of consciousness. Determination conditions are related to at least one of the eye opening/closing feature, the movement feature, and the response feature described above. Individual determination conditions are associated with a determination ID, a determination criterion, and a classification result, for example. The classification results may be two conditional branches indicating "YES/NO", but may also be three or more conditional branches. Also, the determination information 56 and 58 may be provided separately depending on whether a sedative is administered to the subject 12.

The estimation information 60 is information indicating an estimation result from the state estimation unit 40 regarding the state of consciousness, and includes each value of the features, a classification into a level of consciousness, sedation, or the like, a severity score, and an indication of whether notification is necessary, for example.

### [Operations by server apparatus 20]

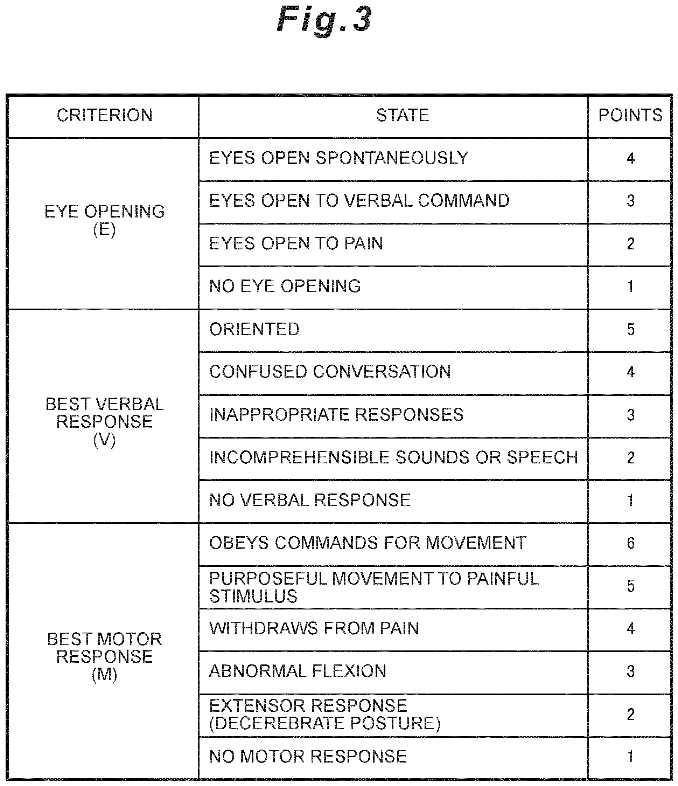

The observation system 10 in the embodiment is configured as above. Next, analysis operations (more specifically, operations for estimating the state of consciousness) by the server apparatus 20 will be described with reference to FIGS. 3 to 11.

FIG. 3 is a diagram illustrating a points table for the GCS, which indicates the severity of consciousness impairment. The GCS includes the three evaluation criteria of [1] eye opening (E score), [2] best verbal response (V score), and [3] best motor response (M score). "Eye opening" is evaluated in four levels from "4", which corresponds to a mild level, to "1", which corresponds to a severe level. "Best verbal response" is evaluated in five levels from "5", which corresponds to a mild level, to "1", which corresponds to a severe level. "Best motor response" is evaluated in six levels from "6", which corresponds to a mild level, to "1", which corresponds to a severe level.

In methods of the related art, a specialist such as a physician or a nurse observes the behavior of the subject 12 and estimates the state of consciousness by assigning points to each evaluation criterion. In this case, there is the problem of an increased burden on the specialist due to observation and evaluation. In contrast, according to the observation system 10, the image data 52 obtained through image capture by the camera 18 can be used to estimate the state of consciousness of the subject 12 automatically, and thus the burden on the specialist can be reduced greatly.

### <Basic operations>

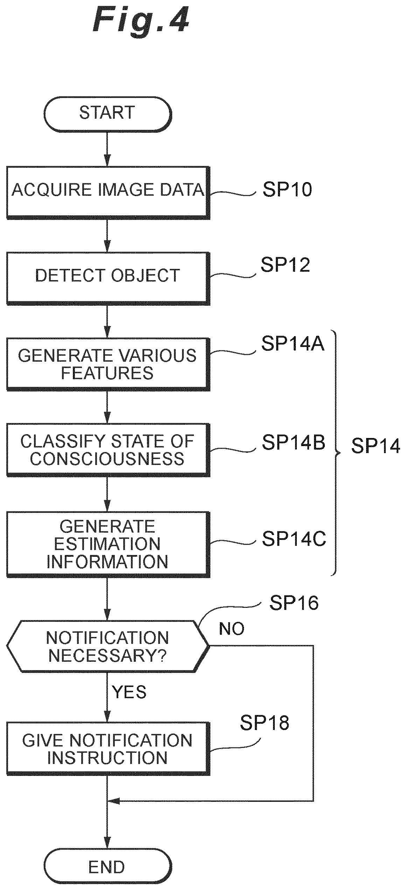

Next, basic operations by the server apparatus 20 in FIGS. 1 and 2 will be described with reference to the flowchart of FIG. 4 and FIGS. 5 to 7.

In step SP10 of FIG. 4, the control unit 32 (more specifically, the image acquisition unit 36) acquires the image data 52 expressing a time series of images obtained by capturing the subject 12 on the bed 16 and the area around the subject 12.

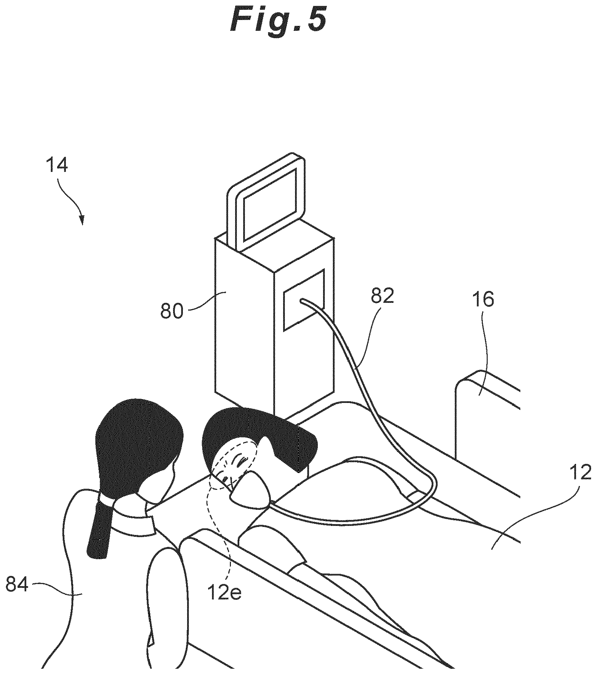

FIG. 5 is a perspective view illustrating an example inside the room 14 in FIG. 1. The subject 12 is lying on the bed 16 provided inside the room 14. In front of the bed 16, medical equipment 80 for causing oxygen to be inhaled into the body of the subject 12 is disposed. The subject 12 is fitted with a tube 82 for introducing oxygen supplied from the medical equipment 80. Also, a medical personnel member 84 checking on the condition of the subject 12 stands beside the bed 16. In the example illustrated in the drawing, the eyes 12e of the subject 12 are directed toward the medical personnel member 84.

The images obtained by capturing the interior of the room 14 include [1] a "first human region" indicating the form of the subject 12, [2] a "bed region" indicating the form of the bed 16, [3] an "equipment region" indicating the form of the medical equipment 80, [4] a "tube region" indicating the form of the tube 82, and [5] a "second human region" indicating the form of the medical personnel member 84. Also, an "eye region" indicating the eyes 12e of the subject 12 is included in a head location of the first human region.

In step SP12 of FIG. 4, the control unit 32 (more specifically, the object detection unit 38) performs an object detection process on the image data 52 acquired in step SP10, and detects [1] the presence/absence, location, and pose of the subject 12, [2] the location and open/closed state of the eyes 12e, [3] the presence/absence, location, and pose of specific objects (for example, the medical equipment 80, the tube 82, and the medical personnel member 84), [4] the presence/absence and degree of response (for example, a temporal change in location and pose) by the subject 12 with respect to the specific objects, and the like.

In step SP14, the control unit 32 (more specifically, the state estimation unit 40) estimates the state of consciousness of the subject 12 on the basis of the detection results in step SP12. Step SP14 includes the three sub-steps of [1] generating various features (step SP14A), [2] classifying the state of consciousness (step SP14B), and [3] generating the estimation information 60 (step SP14C).

In step SP14A, the state estimation unit 40 (more specifically, the first generation unit 44) generates various features (such as the eye opening/closing feature, the movement feature, and the response feature, for example) necessary for the classification process on the basis of the detection results in step SP12.

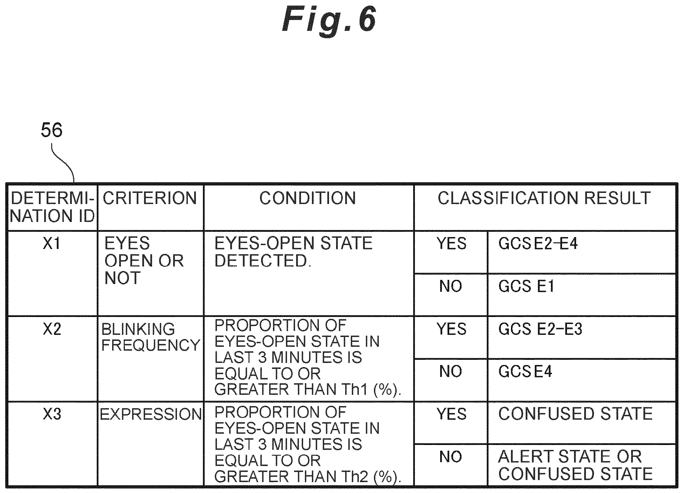

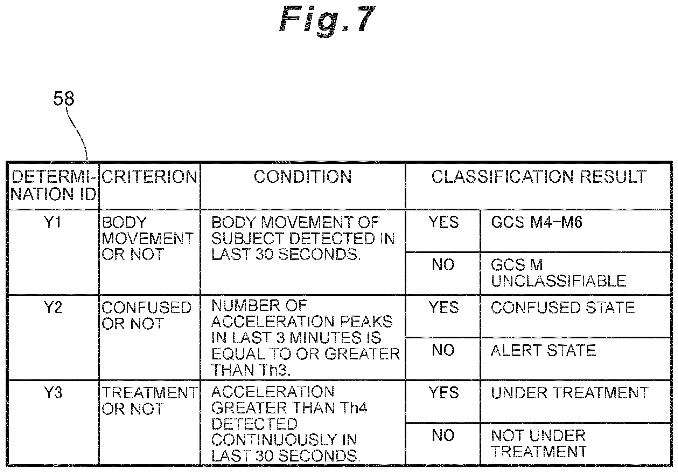

In step SP14B, the state estimation unit 40 (more specifically, the classification unit 46) uses the various features generated in step SP14A to perform a classification process for classifying the state of consciousness of the subject 12. The classification process is performed in accordance with the determination information 56 and 58 illustrated in FIGS. 6 and 7, for example.

FIG. 6 is a table illustrating an example of determination conditions specified by the determination information 56 related to a temporal change in eye opening/closing. In the example illustrated in the drawing, the determination information 56 describes three determination conditions (namely, eye opening/closing determinations X1, X2, X3). The individual determination conditions are associated with a determination ID, a determination criterion, and a classification result, for example.

According to the determination criterion "eyes open or not" with the determination ID "X1" (that is, the eye opening/closing determination X1), the GCS E score is classified into one of "2 to 4" if an eyes-open state is detected, whereas the GCS E score is classified into "1" if an eyes-open state is not detected.

According to the determination criterion "blinking frequency" with the determination ID "X2" (that is, the eye opening/closing determination X2), the GCS E score is classified into one of "2 to 3" if the proportion of time in an eyes-open state (that is, the eyes-open ratio) in the last three minutes is equal to or greater than a threshold value Th1 (units: %), whereas the GCS E score is classified into "4" if the eyes-open ratio falls below Th1.

According to the determination criterion "expression" with the determination ID "X3" (that is, the eye opening/closing determination X3), the subject 12 is classified into the "confused state" if the eyes-open ratio in the last three minutes is equal to or greater than a threshold value Th2 (units: %), whereas the subject 12 is classified into the "alert state" or the "confused state" if the eyes-open ratio falls below Th2.

FIG. 7 is a table illustrating an example of determination conditions specified by determination information 58 related to body movement of the subject 12. In the example illustrated in the drawing, the determination information 58 describes three determination conditions (namely, body movement determinations Y1, Y2, Y3). The individual determination conditions are associated with a determination ID, a determination criterion, and a classification result, for example.

According to the determination criterion "body movement or not" with the determination ID "Y1" (also referred to as the "body movement determination Y1"), the GCS M score is classified into one of "4 to 6" if body movement has been detected in the last 30 seconds, whereas the GCS M score is classified as "undecidable" if body movement is not detected.

According to the determination criterion "confused or not" with the determination ID "Y2" (also referred to as the "body movement determination Y2"), the subject 12 is classified into the "confused state" if the number of acceleration peaks in body movement in the last three minutes is equal to or greater than a threshold value Th3 (units: number), whereas the subject 12 is classified into the "alert state" if the number of peaks falls below Th3.

According to the determination criterion "treatment or not" with the determination ID "Y3" (also referred to as the "body movement determination Y3"), the subject 12 is classified as being "under treatment" if a body movement acceleration greater than a threshold value Th4 (units: mm/s²) has been detected continuously in the last 30 seconds, whereas the subject 12 is classified as being "not under treatment" if the acceleration is not detected continuously.

In step SP14C of FIG. 4, the state estimation unit 40 (more specifically, the second generation unit 48) generates estimation information 60 indicating an estimation result according to the classification in step SP14B. After executing step SP14, the control unit 32 proceeds to the next step SP16.

In step SP16, the control unit 32 (more specifically, the notification instruction unit 42) determines whether external notification is necessary on the basis of the estimation result in step SP14. If notification is determined to be necessary (step SP16: YES), the notification instruction unit 42 proceeds to the next step SP18. On the other hand, if notification is determined to be unnecessary (step SP16: NO), the control unit 32 skips the execution of step SP18 and ends the operations of the flowchart illustrated in FIG. 4.

In step SP18, if notification is determined to be necessary in step SP16, the notification instruction unit 42 causes notification data including the estimation information 60 to be transmitted to a relevant terminal apparatus 22 through the communication unit 30. The terminal apparatus 22, after receiving the notification data, uses its own output function units to produce an external notification. Thereafter, the control unit 32 ends the operations of the flowchart illustrated in FIG. 4.

In this way, by having the server apparatus 20 repeatedly execute the flowchart of FIG. 4 periodically or aperiodically, a medical personnel member can observe the state of consciousness of the subject 12.

### <Specific example of estimation operations>

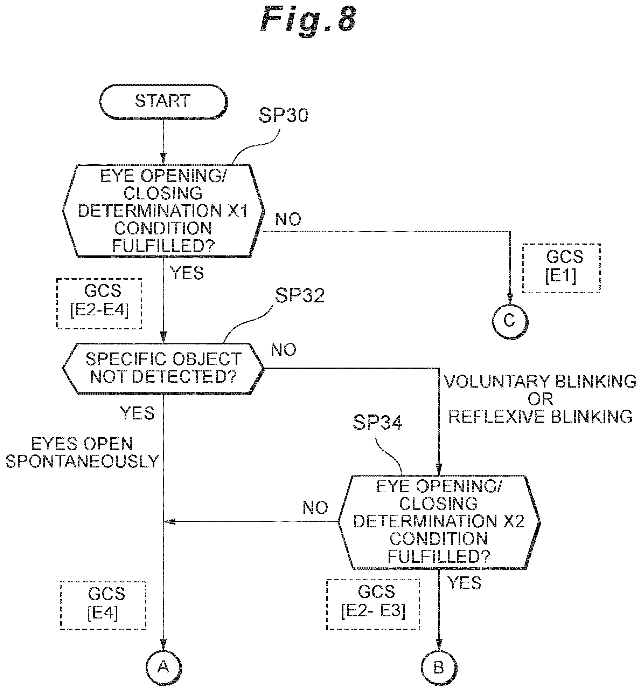

Next, a specific example of estimation operations in the case where the determination information 56 and 58 in FIGS. 6 and 7 is used will be described with reference to the flowcharts of FIGS. 8 to 11. The following describes an example for the case in which a sedative is not administered to the subject 12, or in other words, the subject 12 is not in the "sedated state".

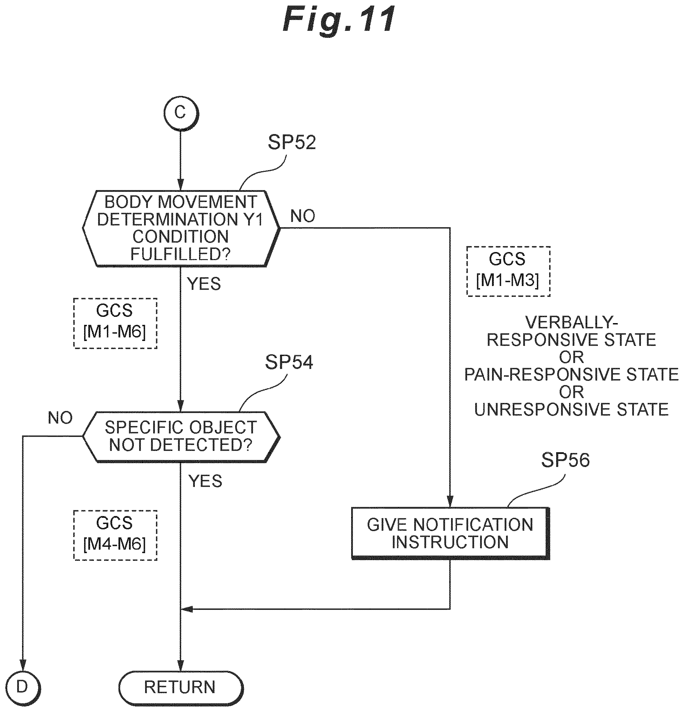

In FIG. 8, the state estimation unit 40 makes the eye opening/closing determination X1 (step SP30). If the given condition is fulfilled (step SP30: YES), the state estimation unit 40 classifies the GCS E score into one of "2 to 4" and proceeds to the following step SP32. On the other hand, if the given condition is not fulfilled (step SP30: NO), the state estimation unit 40 classifies the E score into "1" and proceeds to the flowchart (endpoint C) of FIG. 11 described later.

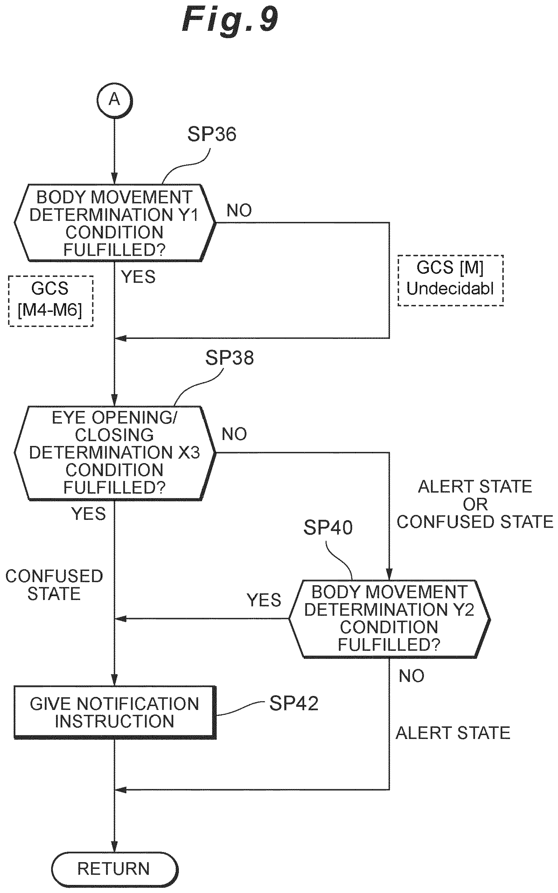

After the primary classification regarding the E score is finished, the state estimation unit 40 determines whether a specific object different from the subject 12 has not been detected (step SP32). If a specific object has not been detected (step SP32: YES), the state estimation unit 40 classifies blinking by the subject 12 as being "spontaneous blinking", classifies the E score into "4", and then proceeds to the flowchart (endpoint A) of FIG. 9 described later. On the other hand, if the given condition is not fulfilled (step SP32: NO), the state estimation unit 40 classifies the blinking by the subject 12 as being "voluntary blinking" or "responsive blinking", and proceeds to the following step SP34.

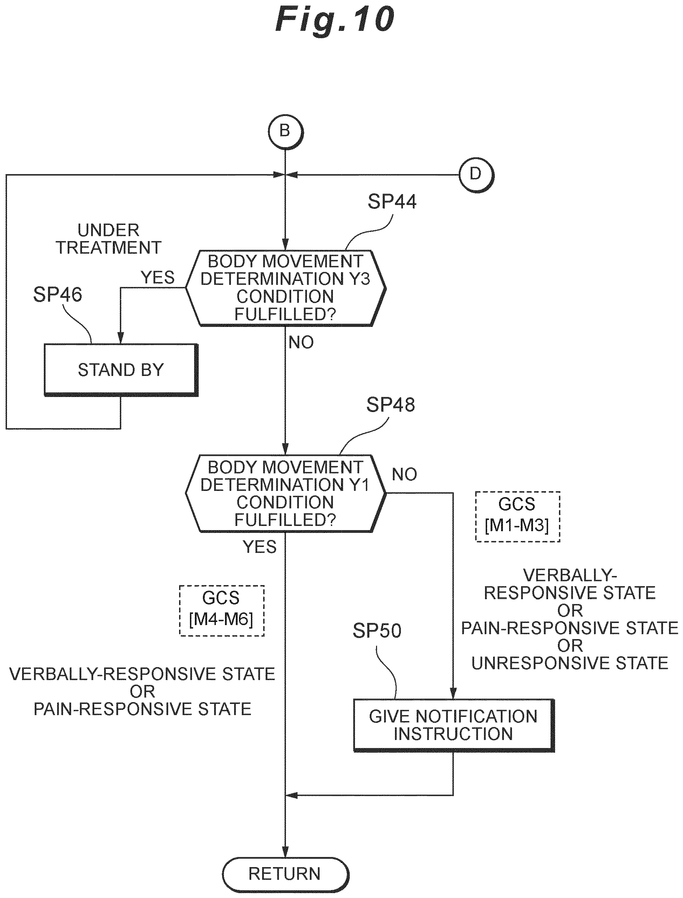

After the classification regarding the type of blinking is finished, the state estimation unit 40 makes the eye opening/closing determination X2 (step SP34). If the given condition is fulfilled (step SP34: YES), the state estimation unit 40 classifies the E score into "4" and proceeds to the flowchart (endpoint B) of FIG. 9 described later. On the other hand, if the given condition is not fulfilled (step SP34: NO), the state estimation unit 40 classifies the E score into one of "2 to 3" and proceeds to the flowchart (endpoint C) of FIG. 10 described later.

In FIG. 9, after the primary classification regarding the E score is finished (refer to endpoint A of FIG. 8), the state estimation unit 40 makes the body movement determination Y1 (step SP36). If the given condition is fulfilled (step SP36: YES), the state estimation unit 40 classifies the GCS M score into one of "4 to 6" and proceeds to the following step SP38. On the other hand, if the given condition is not fulfilled (step SP36: NO), the state estimation unit 40 classifies the M score as being "undecidable" and proceeds to the following step SP38.

After the classification regarding the M score is finished, the state estimation unit 40 makes the eye opening/closing determination X3 (step SP38). If the given condition is fulfilled (step SP38: YES), the state estimation unit 40 classifies the subject 12 into the "confused state" and proceeds to step SP42. On the other hand, if the given condition is not fulfilled (step SP38: NO), the state estimation unit 40 classifies the subject 12 into the "confused state" or the "alert state" and proceeds to step SP40.

Next, the state estimation unit 40 makes the body movement determination Y2 (step SP40). If the given condition is fulfilled (step SP40: YES), the state estimation unit 40 classifies the subject 12 into the "confused state" and proceeds to the next step SP42. On the other hand, if the given condition is not fulfilled (step SP40: NO), the state estimation unit 40 classifies the subject 12 into the "alert state" and ends the operations illustrated in the flowcharts of FIGS. 8 to 11.

If the subject 12 is in the "confused state", the state estimation unit 40 informs the notification instruction unit 42 that a notification instruction should be given (step SP42), and ends the operations illustrated in the flowcharts of FIGS. 8 to 11.

In FIG. 10, after the secondary classification regarding the E score is finished (refer to endpoint B of FIG. 8), the state estimation unit 40 makes the body movement determination Y3 (step SP44). If the given condition is fulfilled (step SP44: YES), the state estimation unit 40 classifies the subject 12 as being "under treatment", and after standing by for a prescribed length of time (step SP46), returns to step SP38. On the other hand, if the given condition is not fulfilled (step SP44: NO), the state estimation unit 40 considers the subject 12 as not being "under treatment" and proceeds to the next step SP48.

If the subject 12 is not being treated, the state estimation unit 40 makes the body movement determination Y1 (step SP48). If the given condition is fulfilled (step SP48: YES), the state estimation unit 40 classifies the GCS M score into one of "4 to 6", classifies the subject 12 as being in the "verbally-responsive state" or the "pain-responsive state", and ends the operations illustrated in the flowcharts. On the other hand, if the given condition is not fulfilled (step SP48: NO), the state estimation unit 40 classifies the subject 12 into one of the "verbally-responsive state", the "pain-responsive state", or the "unresponsive state", and proceeds to the next step SP50.

If there is a possibility that the subject 12 is in the "unresponsive state", the state estimation unit 40 informs the notification instruction unit 42 that a notification instruction should be given (step SP50), and ends the operations illustrated in the flowcharts of FIGS. 8 to 11.

In FIG. 11, after the classification regarding the E score is finished (refer to endpoint C of FIG. 8), the state estimation unit 40 makes the body movement determination Y1 (step SP52). If the given condition is fulfilled (step SP52: YES), the state estimation unit 40 classifies the GCS M score into one of "1 to 6" and proceeds to the following step SP54.

If the M score cannot be classified, the state estimation unit 40 determines whether a specific object different from the subject 12 has not been detected (step SP54). If a specific object has not been detected (step SP54: YES), the state estimation unit 40 classifies the GCS M score into one of "4 to 6" and ends the operations illustrated in the flowcharts. On the other hand, if a specific object has been detected (step SP54: NO), the state estimation unit 40 proceeds to step SP44 in the flowchart (endpoint D) of FIG. 10 described above.

Meanwhile, returning to step SP52, if the given condition is not fulfilled (step SP52: NO), the state estimation unit 40 classifies the GCS M score into one of "1 to 3", classifies the subject 12 into the "verbally-responsive state", the "pain-responsive state", or the "unresponsive state", and proceeds to the next step SP54.

If there is a possibility that the subject 12 is in the "unresponsive state", the state estimation unit 40 informs the notification instruction unit 42 that a notification instruction should be given (step SP54), and ends the operations in the flowcharts of FIGS. 8 to 11. In this way, by having the server apparatus 20 repeatedly execute the flowcharts of FIGS. 8 to 11 periodically or aperiodically, a medical personnel member can observe the state of consciousness of the subject 12.

The above describes an example for the case in which the subject 12 is not in the "sedated state", but the state of consciousness may also be estimated according to a flowchart similar to FIGS. 8 to 11 even in the case in which a sedative has been administered to the subject 12. However, in this case, the rules may be changed as necessary and where appropriate, such as by using the RASS in place of the GCS.

### [Summary of embodiment]

As above, the server apparatus 20 as a state of consciousness analysis apparatus according to the embodiment is provided with: the image acquisition unit 36 that acquires the image data 52 expressing a time series of images obtained by capturing the subject 12 on the bed 16 and the area around the subject 12; the object detection unit 38 that performs an object detection process on the image data 52 acquired by the image acquisition unit 36 to detect a temporal change in eye opening/closing of the subject 12; and the state estimation unit 40 that uses at least the eye opening/closing feature relating to the temporal change in eye opening/closing detected by the object detection unit 38 to estimate the state of consciousness of the subject 12.

Also, according to a state of consciousness analysis method or program according to the embodiment, one or multiple computers (alternatively, one or multiple processors) execute: an acquiring step (SP10) for acquiring the image data 52 expressing a time series of images obtained by capturing the subject 12 on the bed 16 and the area around the subject 12; a detecting step (SP12) for detecting a temporal change in eye opening/closing of the subject 12 on the basis of the acquired image data 52; and an estimating step (SP14) for using at least the eye opening/closing feature relating to the detected temporal change in eye opening/closing to estimate the state of consciousness of the subject 12.

In this way, since the state of consciousness is estimated by using the eye opening/closing feature relating to the detected temporal change in eye opening/closing, the estimation accuracy regarding the state of consciousness of the subject 12 can be raised further under a variety of circumstances that may vary from moment to moment.

Also, the state estimation unit 40 may classify the state of consciousness of the subject 12 into one of a plurality of eye opening levels through multiple varieties of determination processes differing in the determination time length. The eye opening level can be classified in stages through the multiple varieties of determination processes.

The eye opening/closing feature may also include a statistic relating to the frequency or speed of blinking or a statistic indicating the proportion of the eyes-open time or the eyes-closed time. This arrangement makes it possible to analyze, for example, the presence/absence and type of blinking, the facial expression, and the like, and the estimation accuracy regarding the state of consciousness can be raised further.

In the case in which the object detection unit 38 detects body movement of the subject 12, the state estimation unit 40 may also estimate the state of consciousness of the subject 12 by additionally using a movement feature relating to the body movement detected by the object detection unit 38. By combining the eye opening/closing feature and the body movement feature, the estimation accuracy regarding the state of consciousness can be raised further.

Also, the state estimation unit 40 may classify the state of consciousness of the subject 12 into one of a plurality of motor response levels through multiple varieties of determination processes differing in the determination time length. The motor response level can be classified in stages through the multiple varieties of determination processes.

The movement feature may also include a statistic relating to the velocity, acceleration, or distance of body movement or to a specific behavior of the subject 12. This arrangement makes it possible to analyze, for example, the presence/absence of body movement, confusion, treatment, and the like, and the estimation accuracy regarding the state of consciousness can be raised further.

In the case in which the object detection unit 38 detects a specific object near the subject 12 (specifically, the medical equipment 80, the tube 82, the medical personnel member 84, or the like), the state estimation unit 40 may also estimate the state of consciousness of the subject 12 by additionally using a response feature relating to the magnitude of a response by the subject 12 with respect to the specific object detected by the object detection unit 38. By combining the eye opening/closing feature and the response feature, the estimation accuracy regarding the state of consciousness can be raised further.

Moreover, the state estimation unit 40 may estimate the state of consciousness such that the severity is greater to the extent that the response feature is small, or such that the severity is lesser to the extent that the response feature is large. By considering how the magnitude of a response by the subject 12 and the severity of consciousness impairment are highly correlated, the estimation accuracy regarding the state of consciousness can be raised further.

The state estimation unit 40 may also estimate the state of consciousness of the subject 12 by excluding a time frame in which the response feature exceeds a threshold value. By pre-excluding from the calculations a time frame containing the possibility of the subject 12 strongly responding to a specific object, the estimation accuracy regarding the state of consciousness can be raised further.

The state estimation unit 40 may also estimate the state of consciousness of the subject 12 according to different estimation rules depending on whether a sedative is administered to the subject 12. By considering how the behavior of the subject 12 may change depending on whether a sedative is administered, the estimation accuracy regarding the state of consciousness can be raised further.

In addition, an observation system 10 according to the present embodiment is provided with: a camera 18 that outputs image data 52 expressing a time series of images obtained by capturing the subject 12 on the bed 16 and the area around the subject 12; a server apparatus 20 that gives an instruction for external notification on the basis of the image data 52 outputted from the camera 18; and a notification apparatus (herein, the terminal apparatus 22) that produces an external notification according to the notification instruction from the server apparatus 20.

In this case, the server apparatus 20 is provided with a notification instruction unit 42 that determines whether an external notification is necessary on the basis of an estimation result obtained by the state estimation unit 40, and gives a notification instruction in the case where a notification is determined to be necessary. With this arrangement, the user of the terminal apparatus 22 can be informed rapidly about a change in the state of consciousness of the subject 12, as necessary.

### [Modifications]

Note that the present invention is not limited to the foregoing embodiment, and obviously the configuration can be freely modified without departing from the gist of the invention. Moreover, respective configurations may be freely combined in technologically non-contradictory ways. Moreover, the execution sequence of the steps included in the flowcharts may also be changed in technologically non-contradictory ways.

## Claims

1. A state of consciousness analysis apparatus (20) comprising:
an image acquisition unit (36) that acquires image data (52) expressing a time series of images obtained by capturing a subject (12) on a bed (16) and an area around the subject (12);
an object detection unit (38) that performs an object detection process on the image data (52) acquired by the image acquisition unit (36) to detect a temporal change in eye opening/closing of the subject (12); and
a state estimation unit (40) that uses at least an eye opening/closing feature relating to the temporal change in eye opening/closing detected by the object detection unit (38) to estimate a state of consciousness of the subject (12).

2. The state of consciousness analysis apparatus (20) according to claim 1, wherein the state estimation unit (40) classifies the state of consciousness of the subject (12) into one of a plurality of eye opening levels by performing multiple varieties of determination processes differing in a combination of a type of the eye opening/closing feature and a determination time length.

3. The state of consciousness analysis apparatus (20) according to claim 1, wherein the eye opening/closing feature includes a statistic relating to a frequency or a speed of blinking.

4. The state of consciousness analysis apparatus (20) according to claim 1, wherein the eye opening/closing feature includes a statistic indicating a proportion of an eyes-open time or an eyes-closed time.

5. The state of consciousness analysis apparatus (20) according to claim 1 to 4, wherein
the object detection unit (38) additionally detects body movement of the subject (12), and
the state estimation unit (40) estimates the state of consciousness of the subject (12) by additionally using a movement feature relating to the body movement detected by the object detection unit (38).

6. The state of consciousness analysis apparatus according to claim 5, wherein the state estimation unit (40) classifies the state of consciousness of the subject (12) into one of a plurality of motor response levels by performing multiple varieties of determination processes differing in a type of the movement feature and a determination time length.

7. The state of consciousness analysis apparatus (20) according to claim 5, wherein the movement feature includes a statistic relating to a velocity of the body movement, an acceleration of the body movement, a distance of the body movement, or a specific behavior of the subject (12).

8. The state of consciousness analysis apparatus (20) according to claim 1 to 7, wherein
the object detection unit (38) additionally detects a specific object (80, 82, 84) near the subject (12), and
the state estimation unit (40) estimates the state of consciousness of the subject (12) by additionally using a response feature relating to a magnitude of a response by the subject (12) with respect to the specific object (80, 82, 84) detected by the object detection unit (38) .

9. The state of consciousness analysis apparatus (20) according to claim 8, wherein the state estimation unit (40) estimates the state of consciousness such that a severity is greater to the extent that the response feature is small, or such that the severity is lesser to the extent that the response feature is large.

10. The state of consciousness analysis apparatus (20) according to claim 8 or 9, wherein the state estimation unit (40) estimates the state of consciousness of the subject (12) by excluding a time frame in which the response feature exceeds a threshold value.

11. The state of consciousness analysis apparatus (20) according to claim 1 to 10, wherein the state estimation unit (40) estimates the state of consciousness of the subject (12) according to different rules depending on whether a sedative is administered to the subject (12).

12. The state of consciousness analysis apparatus (20) according to claim 1 to 11, further comprising:
a notification instruction unit (42) that determines whether an external notification is necessary on a basis of an estimation result obtained by the state estimation unit (40), and gives a notification instruction in a case where the notification is determined to be necessary.

13. A state of consciousness analysis program causing one or multiple computers (20) to execute:
an acquiring step of acquiring image data (52) expressing a time series of images obtained by capturing a subject (12) on a bed (16) and an area around the subject (12);
a detecting step of detecting a temporal change in eye opening/closing of the subject (12) on a basis of the acquired image data (52); and
an estimating step of using at least an eye opening/closing feature relating to the detected temporal change in eye opening/closing to estimate a state of consciousness of the subject (12).

14. An observation system (10) comprising:
a camera (18) that outputs image data (52) expressing a time series of images obtained by capturing a subject (12) on a bed (16) and an area around the subject (12);
a state of consciousness analysis apparatus (20) that gives an instruction for external notification on a basis of the image data (52) outputted from the camera (18); and
a notification apparatus (22) that produces an external notification in response to the notification instruction from the state of consciousness analysis apparatus (20), wherein
the state of consciousness analysis apparatus (20) comprises:
an image acquisition unit (36) that acquires the image data (52) outputted by the camera (18);
an object detection unit (38) that performs an object detection process on the image data (52) acquired by the image acquisition unit (36) to detect a temporal change in eye opening/closing of the subject (12);
a state estimation unit (40) that uses at least an eye opening/closing feature relating to the temporal change in eye opening/closing detected by the object detection unit (38) to estimate a state of consciousness of the subject (12); and
a notification instruction unit (42) that determines whether an external notification is necessary on a basis of an estimation result obtained by the state estimation unit (40), and gives a notification instruction in a case where the notification is determined to be necessary.
